# EUROPÄISCHE PATENTSCHRIFT

(11) **EP 0 749 724 B1**
(45) Veröffentlichungstag und Bekanntmachung des Hinweises auf die Patenterteilung: **24.02.1999**
(21) Anmeldenummer: 96104943.4
(22) Anmeldetag: 28.03.1996
(51) Int. Cl.: A61B 17/02

(54) **Chirurgischer Wundsperrer**
Surgical retractor
Rétracteur chirurgical

(30) Priorität: 23.06.1995 DE 19522879
(43) Veröffentlichungstag der Anmeldung: 27.12.1996
(73) Patentinhaber: Aesculap AG & Co. KG, 78532 Tuttlingen (DE)
(72) Erfinder: Mayer, Heinz Michael, Priv. Doz. Dr. med., 14195 Berlin (DE); Eckhof, Stephan, Dipl.-Ing. (FH), 78532 Tuttlingen (DE); Giordano, Nicola, Dipl.-Ing. (FH), 78056 Villingen-Schwenningen (DE); Weisshaupt, Dieter, Dipl.-Ing. (FH), 78194 Immendingen (DE)
(74) Vertreter: Böhme, Ulrich, Dr. Dipl.-Phys.

(56) Entgegenhaltungen:
- EP-A- 0 327 249
- FR-A- 2 692 468
- US-A- 4 867 139
- US-A- 5 363 841

## Beschreibung

Die Erfindung betrifft einen chirurgischen Wundsperrer.

Wundsperrer werden benutzt, um bei chirurgischen Eingriffen Muskelgewebe, Gefäße und andere Gewebeteile mit Hilfe von Valven oder Wundsperrerblättern zu repositionieren und damit einen freien Zugang zu dem Operationsgebiet zu schaffen.

Es ist bekannt, ringförmige Rahmen zu verwenden, an denen mehrere Wundsperrerblätter verschiebbar gelagert werden, die jeweils an zu repositionierende Gewebeteile angelegt werden. Eine solche chirurgische Wundsperrervorrichtung wird also am Körper schwimmend gelagert, die Abstützkräfte sind daher bei allen Wundsperrerblättern gleich. Eine individuelle Dosierung dieser Kräfte ist mit vorbekannten Wundsperrern nicht möglich, obwohl dies an sich sehr wünschenswert wäre, da die repositionierten Gewebeteile unterschiedlich empfindlich auf Druck reagieren.

Es ist auch ein Wundsperrer bekannt, der mit einem zangenartigen Instrument verbunden ist und mit diesem an einem Knochen festgelegt werden kann (US-A 4 867 139). Mit dem zangenartigen Instrument ist ein Stab verbunden, an dem zwei Rückhalteblätter aufgehängt werden können, die mit ihren distalen Enden am Knochen festgelegt werden können. Bei einem solchen Instrument ist man hinsichtlich der Positionierung eines Wundblattes sehr beschränkt.

Ausgehend von diesem Stand der Technik ist es Aufgabe der Erfindung, einen chirurgischen Wundsperrer derart auszugestalten, daß eine individuelle Einstellung der Kräfte und eine sichere Positionierung der Wundsperrerblätter möglich ist.

Diese Aufgabe wird gemäß der Erfindung gelöst durch einen chirurgischen Wundsperrer mit einem in sich geschlossenen Rahmen, der mindestens zwei Wundsperrerelemente auf einander gegenüberliegenden Seiten des Rahmens und senkrecht von ihm abstehend zwei Stützfüße mit Befestigungsmitteln zur Festlegung an einem Knochen trägt.

Diese Stützfüße werden bei chirurgischen Eingriffen mittels Befestigungsmitteln an einem Knochen festgelegt, beispielsweise an den Wirbelkörpern der Wirbelsäule, und dadurch wird der Rahmen gegenüber dem Skelett des Patienten in eine definierte Position gebracht. Die zwei Stützfüße können beispielsweise in einem solchen Abstand am Rahmen gehalten werden, daß die Befestigungsmittel der beiden Stützfüße an verschiedenen Wirbelkörpern festgelegt sind. Die Wundsperrerblätter können durch entsprechende Verschiebung am Rahmen mit unterschiedlichen Andruckkräften an die einzelnen Gewebeteile angelegt werden, da der Rahmen starr mit dem Skelett des Patienten verbunden ist und nicht schwimmend allein durch die auf die Wundsperrerblätter ausgeübten Kräfte gelagert wird.

Besonders vorteilhaft ist es, wenn die Befestigungsmittel Knochenschrauben umfassen.

Insbesondere können die Knochenschrauben Verlängerungen tragen, an welchen die Stützfüße gehalten sind.

An sich sind Knochenschrauben mit Verlängerungen, die in Knochen, insbesondere Wirbelkörper, einschraubbar sind, bekannt. So gibt es Instrumente, die über derartige in Wirbelkörper eingeschraubte Knochenschrauben mit Verlängerungen die Relativposition von zwei Wirbelkörpern verändern und gegebenenfalls korrigieren. Diese veränderte Wirbelposition kann dann durch geeignete Vorrichtungen, die an den Knochenschrauben und den Verlängerungen angreifen, fixiert werden, so daß die Wirbelkörper in der geänderten Position über einen längeren Heilungszeitraum festgelegt werden können.

Derartige an sich bekannte Vorrichtungen aus Knochenschraube und Verlängerung werden im Rahmen der vorliegenden Erfindung als Teil eines chirurgischen Wundsperrers eingesetzt und dienen als Befestigungsmittel für den Rahmen eines solchen Wundsperrers.

Insbesondere kann vorgesehen sein, daß die Stützfüße die Verlängerungen aufnehmende Bohrungen aufweisen.

Damit können die Stützfüße in einfacher Weise dadurch an der Wirbelsäule oder an einem anderen Knochenteil festgelegt werden, daß sie auf die Verlängerungen aufgesetzt werden, dabei treten die Verlängerungen in die aufnebmenden Bohrungen ein. Weil zwei derartige Stützfüße verwendet werden, ist der Rahmen allein durch dieses Aufschieben relativ zum Körper verdrehungs- und verschiebungsfrei gehalten, zumindest in einer Ebene senkrecht zu den Verlängerungen.

Es kann zusätzlich vorgesehen sein, daß die Stützfüße Wundsperrerblätter tragen oder selbst als Wundsperrerblätter ausgebildet sind. Die Stützfüße selber wirken somit auch als Anlagefläche für umgebendes Gewebe, zusätzlich erfolgt aber auch noch eine Reposition von Gewebeteilen durch Wundsperrerblätter am Rahmen.

Günstig ist es, wenn die Stützfüße winkelig miteinander verbundene Abschnitte aufweisen und wenn ein Abschnitt Befestigungsmittel und der andere den Rahmen trägt. Dies ist insbesondere dann vorteilhaft, wenn die den Rahmen tragenden Abschnitte in einer Ebene liegen, die parallel zum Rahmen angeordnet ist, so daß sich durch eine Verschiebung längs dieser Abschnitte eine Reposition des Rahmens erreichen läßt.

Allgemein ist vorgesehen, daß der Rahmen an den Stützfüßen verstellbar gehalten ist, so daß der Rahmen in die gewünschte Position relativ zu dem den Rahmen haltenden Knochen gebracht werden kann.

Die nachfolgende Beschreibung einer bevorzugten Ausführungsform der Erfindung dient im Zusammenhang mit der Zeichnung der näheren Erläuterung. Es zeigen:

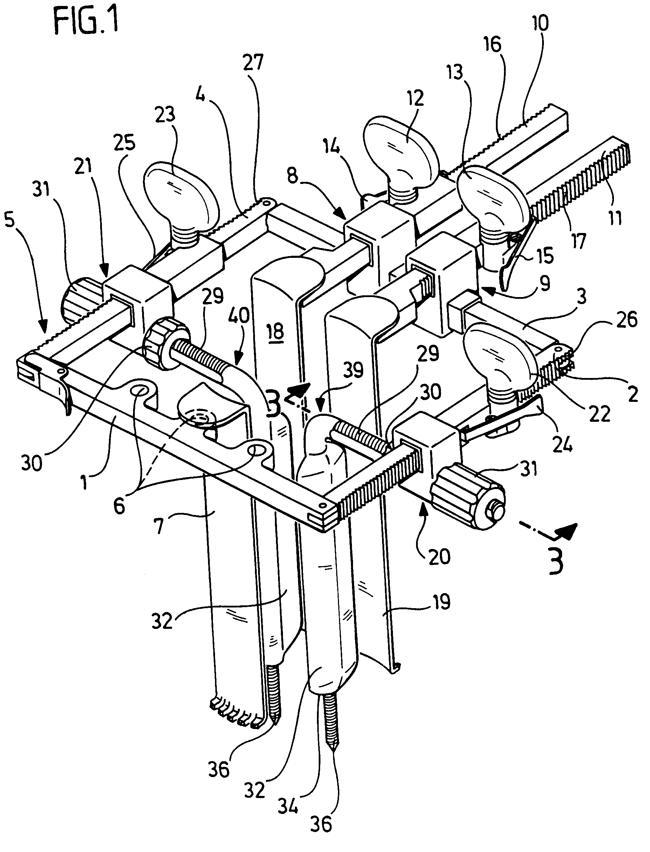

- Figur 1:: eine perspektivische Ansicht eines chirurgischen Wundsperrers mit zwei Stützfüßen und drei verschiebbaren Wundsperrerblättern;

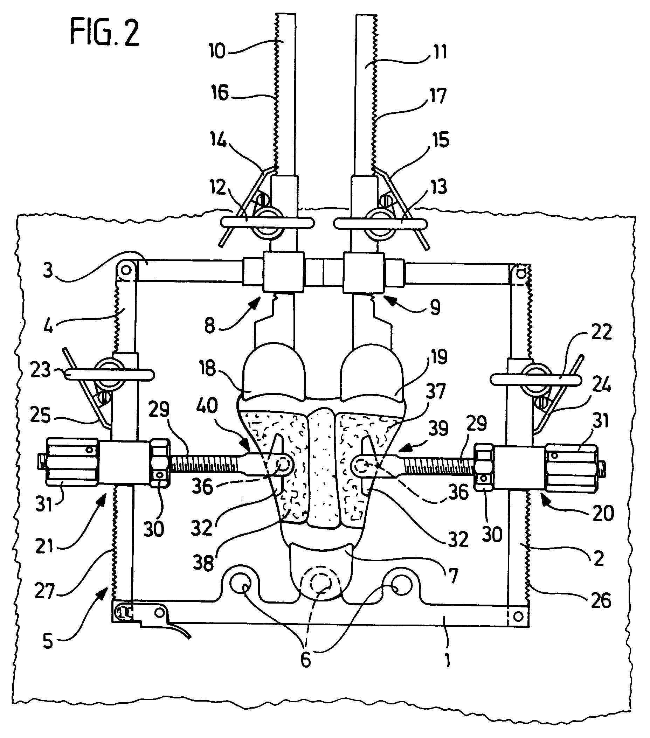

- Figur 2:: eine Draufsicht auf den chirurgischen Wundsperrer der Figur 1 mit an Gewebeteilen anliegenden Wundsperrerblättern und an Knochenteilen des Patienten gehaltenen Stützfüßen und

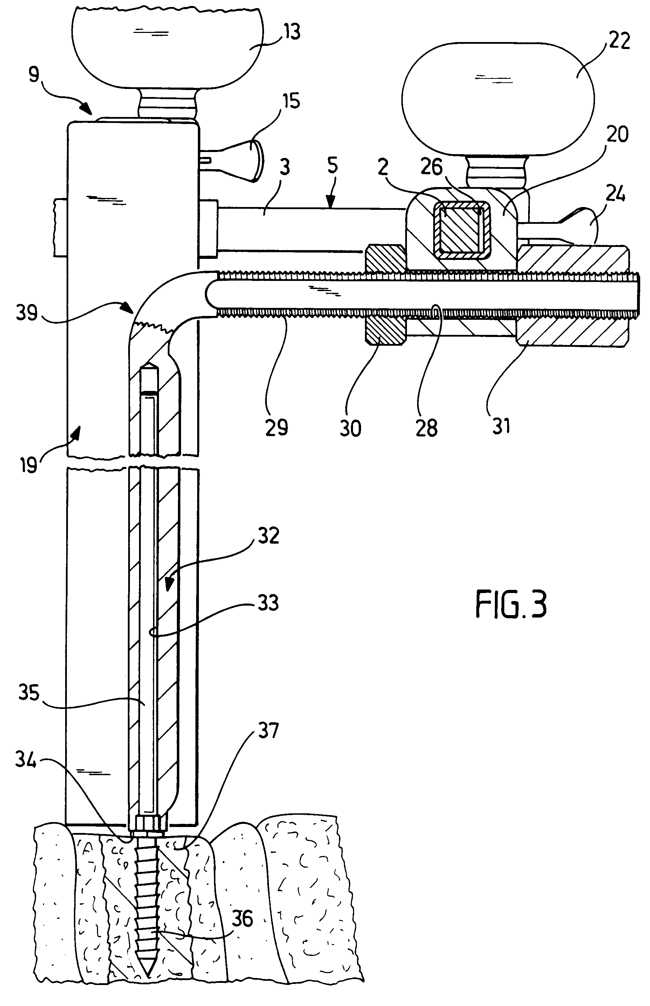

- Figur 3:: eine Schnittansicht längs Linie 3-3 in Figur 1.

Der in der Zeichnung dargestellte Wundsperrer umfaßt vier Arme 1, 2, 3, 4, die zu einem quadratischen Rahmen 5 zusammengefügt sind.

An einem Arm 1 sind Einsteckösen 6 für ein Wundsperrerblatt 7 angeordnet, in die wahlweise Wundsperrerblätter eingesetzt werden können, im dargestellten Ausführungsbeispiel ist lediglich ein solches Wundsperrerblatt 7 in eine der Einsteckösen 6 eingesetzt. Die Wundsperrerblätter 7 können um die Längsachse der Öffnung der Einstecköse 6 verdrehbar sein.

Auf dem gegenüberliegenden Arm 3 sind an dem dargestellten Ausführungsbeispiel zwei Halterungen 8, 9 längsverschieblich gelagert, die durch in der Zeichnung nicht ersichtliche Mittel in einer bestimmten Position längs des Armes 3 festgelegt werden können, beispielsweise durch eine Klemmschraube.

An jeder Halterung 8, 9 ist ein Haltearm 10 bzw. 11 in einer parallel zum Rahmen 5 verlaufenden Ebene und senkrecht zum Arm 3 verschiebbar gelagert, durch Klemmschrauben 12, bzw. 13 sind die Haltearme 10, 11 in den Halterungen 8, 9 festklemmbar, so daß sie nur bei gelösten Klemmschrauben 12, 13 verschoben werden können. Zusätzlich sind jeder Halterung 8, 9 federnde Rasten 14, 15 zugeordnet, die in eine Zahnung 16 bzw. 17 an den Haltearmen 10 und 11 eingreifen. Die Haltearme 10 und 11 können daher bei gelösten Klemmschrauben 12 und 13 nur frei verschoben werden, wenn auch die Rasten 14, 15 gelöst sind, dadurch ist eine Schnellfeststellung bei der Justierung möglich, durch die Klemmschrauben 12, 13 wird diese Stellung dann noch zusätzlich gesichert.

Jeder Haltearm 10, 11 trägt an seinem freien Ende ein Wundsperrerblatt 18 bzw. 19, die ebenso wie das Wundsperrerblatt 7 senkrecht von der durch den Rahmen 5 aufgespannten Ebene abstehen.

Auf den beiden anderen Armen 2, 4 ist jeweils eine Halterung 20 bzw. 21 frei verschieblich gelagert, die durch Klemmschrauben 22 bzw. 23 gegenüber den Armen 2 bzw. 4 festgelegt werden kann. Jeder Halterung 20, 21 ist außerdem eine elastische Raste 24 bzw. 25 zugeordnet, die mit Zahnungen 26 bzw. 27 an den Armen 2 und 4 zusammenwirkt, so daß bei gelösten Klemmschrauben 22, 23 die Halterungen 20 und 21 durch die Rasten 24 und 25 in verschiedenen Positionen vorfixiert werden können.

Jede Halterung 20, 21 weist eine quer zu den Armen 2 bzw. 4 verlaufende Durchgangsbohrung 28 auf, durch die eine Gewindespindel 29 hindurchgesteckt ist. Diese Gewindespindel 29 ist durch zwei auf sie aufgeschraubte Muttern 30, 31, die an gegenüberliegenden Seiten an den Halterungen 20 bzw. 21 anliegen, in axialer Richtung festgelegt, durch Verdrehen der Muttern 30 und 31 ist die Relativposition der Gewindespindel 29 gegenüber diesen Halterungen verstellbar.

Die sich parallel zur Ebene des Rahmens 5 und quer zu den Armen 2 bzw. 4 erstreckende Gewindespindel 29 geht in einen senkrecht von der Ebene des Rahmens 5 abstehenden Wundsperrerkörper 32 über, der ähnlich einem Wundsperrerblatt aber so dick ausgebildet ist, daß er eine Sacklochbohrung 33 aufnehmen kann, die sich von seiner unteren Stirnfläche 34 bis in den oberen Bereich des Wundsperrerkörpers 32 erstreckt.

Die Sacklochbohrung 33 dient der Aufnahme einer stabförmigen Verlängerung 35 einer Knochenschraube 36. Diese Verlängerung hat einen Außendurchmesser, der dem Innendurchmesser der Sacklochbohrung 33 entspricht, so daß die Verlängerung 35 im wesentlichen spielfrei in der Sacklochbohrung 33 aufgenommen werden kann.

Beim Einsatz des beschriebenen Wundsperrers werden zunächst zwei Knochenschrauben 36 mit ihren Verlängerungen 35 in den Knochen des Patienten eingeschraubt, beispielsweise in benachbarte Wirbelkörper 37, 38, und zwar derart, daß die Verlängerungen 35 parallel zueinander verlaufen.

Anschließend wird der beschriebene Wundsperrer so von oben her an die Verlängerungen 35 herangeführt, daß beide Verlängerungen 35 der beiden Knochenschrauben 36 in entsprechende Sacklochbohrungen 33 der beiden Stützfüße 39, 40 eintauchen, die jeweils durch die Gewindespindel 29 und den daran angeformten Wundsperrerkörper 32 gebildet werden. Dies erfolgt bei gelösten Klemmschrauben 22 und 23 und auch bei der frei in den Halterungen 20, 21 verschiebbaren Gewindespindel 29, also vor dem Andrehen der Muttern 30 und 31 an die jeweilige Halterung 20 oder 21.

Grundsätzlich wäre es auch möglich, die Stützfüße 39 und 40 getrennt vom Rahmen 5 auf die Verlängerungen 35 aufzustecken und erst anschließend den Rahmen 5 an den Stützfüßen 39 und 40 zu befestigen, beispielsweise durch Aufstecken der Halterungen 20 und 21.

Der Rahmen 5 kann gegenüber den Stützfüßen 39 und 40 durch die Klemmschrauben 22 und 23 und durch die Muttern 30 und 31 in der gewünschten Relativposition festgelegt werden, so daß praktisch eine starre Verbindung des Rahmens 5 mit den Wirbelkörpern 37 und 38 zu erreichen ist.

Schließlich lassen sich die Wundsperrerblätter 18 und 19 gegenüber dem Rahmen 5 in die gewünschte Position verschieben, so daß ausgehend von dem starr mit den Wirbelkörpern 37 und 38 verbundenen Rahmen 5 die Wundsperrerblätter in die gewünschten Positionen zu verschieben sind und mit der gewünschten Kraft an den zu repositionierenden Geweben anliegen.

Die Vorrichtung kann im übrigen auch verwendet werden, um durch Verstellung der Muttern 30 und 31 den Abstand der beiden Stützfüße 39 und 40 zu ändern und damit die Wirbelkörper 37 und 38 zu repositionieren, falls dies gewünscht wird. Man erhält somit eine Doppelfunktion des Wundsperrers, der auch als Vorrichtung zur Repositionierung der Wirbelkörper eingesetzt werden kann.

## Patentansprüche

1. Chirurgischer Wundsperrer mit einem in sich geschlossenen Rahmen (5), der mindestens zwei Wundsperrerelemente (7, 18, 19; 32) auf einander gegenüberliegenden Seiten des Rahmens (5) und senkrecht von ihm abstehend zwei Stützfüße (39, 40) mit Befestigungsmitteln (35, 36) zur Festlegung an einem Knochen (37, 38) trägt

2. Wundsperrer nach Anspruch 1, dadurch gekennzeichnet, daß die Befestigungsmittel Knochenschrauben (36) umfassen.

3. Wundsperrer nach Anspruch 2, dadurch gekennzeichnet, daß die Knochenschrauben (36) Verlängerungen (35) tragen, an welchen die Stützfüße (39, 40) gehalten sind.

4. Wundsperrer nach Anspruch 3, dadurch gekennzeichnet, daß die Stützfüße (39, 40) die Verlängerungen (35) aufnehmenden Bohrungen (33) aufweisen.

5. Wundsperrer nach einem der voranstehenden Ansprüche, dadurch gekennzeichnet, daß die Stützfüße (39, 40) Wundsperrerblätter (32) tragen.

6. Wundsperrer nach einem der Ansprüche 1 bis 4, dadurch gekennzeichnet, daß die Stützfüße (39, 40) als Wundsperrerblätter (32) ausgebildet sind.

7. Wundsperrer nach einem der voranstehenden Ansprüche, dadurch gekennzeichnet, daß die Stützfüße (39, 40) winkelig miteinander verbundene Abschnitte (29, 32) aufweisen und daß ein Abschnitt (32) die Befestigungsmittel (35, 36) und der andere Abschnitt (29) den Rahmen (5) trägt.

8. Wundsperrer nach einem der voranstehenden Ansprüche, dadurch gekennzeichnet, daß der Rahmen (5) an den Stützfüßen (39, 40) verstellbar gehalten ist.

## Claims

1. A surgical retractor comprising a closed frame (5) carrying at least two surgical retractor components (7, 18, 19; 32) on opposing sides of the frame (5) and, projecting perpendicularly therefrom, two supporting feet (39, 40) having fastening means (35, 36) for fixing to a bone (37, 38).

2. A surgical retractor according to claim 1, characterised in that the fastening means comprise bone screws (36).

3. A surgical retractor according to claim 2, characterised in that the bone screws (36) carry extensions (35) on which the supporting feet (39, 40) are held.

4. A surgical retractor according to claim 3, characterised in that the supporting feet (39, 40) have bores (33) which receive the extensions (35).

5. A surgical retractor according to any one of the preceding claims, characterised in that the supporting feet (39, 40) carry surgical retractor plates (32).

6. A surgical retractor according to any one of claims 1 to 4, characterised in that the supporting feet (39, 40) are formed as surgical retractor plates (32).

7. A surgical retractor according to any one of the preceding claims, characterised in that the supporting feet (39, 40) have portions (29, 32) connected at an angle to one another, and in that one portion (32) carries the fastening means (35, 36) and the other portion (29) carries the frame (5).

8. A surgical retractor according to any one of the preceding claims, characterised in that the frame (5) is adjustably held on the supporting feet (39, 40).

## Revendications

1. Rétracteur chirurgical comportant un cadre (5) refermé sur lui-même et portant au moins deux éléments rétracteurs (7, 18, 19 ; 32) sur des côtés mutuellement opposés du cadre (5) et deux pieds de soutien (39, 40) perpendiculairement en saillie du cadre avec des moyens de fixation (35, 36) pour la fixation sur un os (37, 38).

2. Rétracteur chirurgical selon la revendication 1, caractérisé en ce que les moyens de fixation comprennent des vis à os (36).

3. Rétracteur chirurgical selon la revendication 2, caractérisé en ce que les vis à os (36) portent des prolongements (35) sur lesquels sont retenus les pieds de soutien (39, 40).

4. Rétracteur chirurgical selon la revendication 3, caractérisé en ce que les pieds de soutien (39, 40) présentent des perçages (33) recevant les prolongements (35).

5. Rétracteur chirurgical selon l'une quelconque des revendications précédentes, caractérisé en ce que les pieds de soutien (39, 40) portent des palettes rétractrices (32).

6. Rétracteur chirurgical selon l'une quelconque des revendications 1 à 4, caractérisé en ce que les pieds de soutien (39, 40) sont réalisés sous forme de palettes rétractrices (32).

7. Rétracteur chirurgical selon l'une quelconque des revendications précédentes, caractérisé en ce que les pieds de soutien (39, 40) comportent des tronçons (29, 32) reliés en équerre l'un à l'autre, et en ce qu'un tronçon (32) porte les moyens de fixation (35, 36) et l'autre tronçon (29) porte le cadre (5).

8. Rétracteur chirurgical selon l'une quelconque des revendications précédentes, caractérisé en ce que le cadre (5) est retenu de façon réglable sur les pieds de soutien (39, 40).
